(19) Europäisches Patentamt

European Patent Office

Office européen des brevets

(11) EP 0 898 178 A2

## (12) EUROPEAN PATENT APPLICATION

(43) Date of publication:
24.02.1999 Bulletin 1999/08

(21) Application number: 98200224.8

(22) Date of filing: 26.01.1998

(51) Int. Cl.⁶: $G01T\ 1/164$

(84) Designated Contracting States:
AT BE CH DE DK ES FI FR GB GR IE IT LI LU MC NL PT SE
Designated Extension States:
AL LT LV MK RO SI

(30) Priority: 19.08.1997 EP 97202538

(71) Applicant: van Dulmen, A.A. Dr.
1871 CZ Schoorl (NL)

(72) Inventor:
The designation of the inventor has not yet been filed

(54) Method of imaging by SPECT

(57) The present invention relates to a method of imaging a target organ in a patient by SPECT, by using a gamma camera having a gamma detector provided with a fan-beam collimator, focusing to a focal line parallel to the patient's body length, and by computer reconstructing the distribution of the radioactivity inside the patient's body from the acquired planar images by using certain reconstruction algorithm. The images are acquired along at least one linear orbit performed in a direction perpendicular to the patient's body, and the collimator focal line in made to travel throughout said target organ during the acquisition.

The invention further relates to an equipment for performing this method.

fig.1

Printed by Xerox (UK) Business Services
2.16.7/3.6

## Description

The invention relates to a method of imaging a target organ in a patient by SPECT, by using a gamma camera having a gamma detector provided with a fan-beam collimator, focusing to a focal line parallel to the patient's body length, and by computer reconstructing the distribution of the radioactivity inside the patient's body from the acquired planar images by using certain reconstruction algorithms.

The Single Photon Emission Computed Tomography (SPECT) is routinely used in clinical studies. SPECT is performed by using a gamma camera, comprising a collimator fixed on a gamma detector, which gamma camera follows a revolution orbit around the patient's body. The gamma rays, emitted by a radioactive tracer, accumulated in certain tissues or organs of the patient's body, are sorted by the collimator and recorded by the gamma detector under various angles around the body, the collimator always pointing to (facing) the rotation axis of the camera. From the acquired planer images the distribution of the activity inside the patient's body can be computed using certain reconstruction algorithms. Generally the so-called Expectation-Maximization of the Maximum-Likelihood (EM-ML) algorithm is used, as described by Shepp et. al. (IEEE Trans. Med. Imaging 1982; 2: 113-122) and by Lange et al. (J. Comput. Assist. Tomogr. 1984: 8:306-316). This iterative algorithm minimizes the effect if noise in SPECT images.

The collimators nowadays in use are manufactured from a lead sheat perforated with a plurality of usually parallel holes. The collimator is the most problematic element of the SPECT device, with regard to its poor sensitivity (less than 0.01% of the gamma radiation passed the collimator and reaches the detector) and its poor spatial resolution, becoming increasingly worse with increasing distance between activity source (i.e. the organ or tissue wherein the radioactivity has been accumulated) and collimator. Improvement of one of these properties, e.g. by modifying the hole length or diameter of the collimator, is always to the detriment of the other one. Furthermore, the SPECT technique is inadequate in producing reliable images because of the fact that small fluctuations in the acquired data can involve significant variations in the reconstructed images. This is due to the geometry of the acquired data. The limited time available for obtaining the necessary information (because of the restricted fixation time of the patient and the decay time of the radioactive tracer) and the limited injected radioactivity dose (limited for health care reasons) lead to acquired images containing statistical noise. Indeed the measurement of a radioactive process follows the Poisson law, giving a signal to noise ratio proportional to the count rate. As a result, the reconstructed images are frequently corrupted by significant false positive information, so-called noise artefacts. Consequently, it is a major goal in SPECT imaging to increase the SPECT sensitivity without reduction of the spatial resolution in order to improve the acquired signal to noise ratio.

In an attempt to improve the sensitivity-resolution couple of the collimator, fan-beam collimators, focusing to a focal line, have been developed recently: see e.g. the review articles by Moore et al. (Eur. J. Nucl. Med. 1992; 19:138-150) and by Rosenthal et al. (J. Nucl. Med. 1995; 36:1489-1513). These collimators, having holes converging in one dimension to a focal line, have an increasingly better sensitivity-resolution couple when the activity source approaches the collimator focal line. By using a fan-beam collimator in the SPECT imaging technique, acquiring the images along the classical revolution orbit, the focal line is parallel to the axis of rotation of the gamma camera on the other side of the patient and consequently parallel to the patient's body length (see the above publication by Rosenthal et al., p.1495). Nevertheless, the activity source, i.e. the target organ, has only a restricted approaching range with regard to the collimator focal line, because said organ and all activity contained in the same transverse (i.e. perpendicular to the patient's length) slice must be kept within the collimator acceptance angle during the acquisition by the rotating camera. Otherwise, the reconstructed images are corrupted by significant truncature artefacts. This problem of image truncation by using fan-beam collimators is discussed in more detail by Manglos et al. (Phys. Med. Biol. 1993; 38:1443-1457) and by Kadrmas et al. (Phys. Me.Biol. 1995; 40:1085-1101). The above requirement, viz. to keep all source activity, i.e. in fact the complete body diameter of the patient, within the collimator acceptance angle during the acquisition along a revolution orbit, limits the choice of fan-beam collimators to those having a relatively large focal length, viz. greater than approx. 60 cm, giving results not very different from those obtained with a parallel collimator. Therefore, the target organ cannot be positioned close to the focal line of the collimator where its sensitivity and spatial resolution are optimal. As a consequence, the sensitivity improvement obtained by this technique is limited to a factor of approx. 1.5 at most. Also the target of interest must be smaller that the detector transverse slice (preferably approx. 1.4 times smaller).

It is the objective of the present invention to provide a method of imaging by SPECT with a considerably improved sensitivity-resolution couple. In other words, it is the aim of the present invention to provide a method of SPECT imaging which results in substantially improved reconstructed images.

This objective can be achieved by a method as defined in the opening paragraph, viz. a method of SPECT imaging a target organ in a patient, by using a gamma camera having a gamma detector provided with a fan-beam collimator, focusing to a focal line parallel to the patient's body, followed by computer reconstruction of the radioactivity distribution from the acquired images, which method according to the present invention is characterized in that the images are acquired along at least one linear orbit performed in a direction perpendicular to the patient's body, and in that the collimator focal line is made to travel throughout said target organ during the acquisition.

It has surprisingly been found, that by applying the above method, wherein the usable transverse size dimension of the SPECT device can now be fully used (i.e. the target organ size has now only to be equal at most to the detector transverse size, because the target organ has no longer to be kept within the collimator acceptance angle during the acquisition), the acquired set of planar images is complete (i.e. sufficient to reconstruct the activity distribution) and that considerable improvements with regard to the sensitivity-resolution couple can be obtained. The advantages will be evident. Better reconstructed images can be obtained by using the same acquisition time and the same dose of injected radioactivity. In this manner lesions or other malignancies in the body of a patient can be detected earlier, for example, metastasation of tumours in an early stage of development. At choice, however, the acquisition time can be reduced considerably to obtain, with the same dose of injected radioactivity, images suitable for routine investigations. This results in a reduction of the costs for the clinic or hospital. Also at choice, as a third alternative the dose of injected radioactivity can be reduced in order to burden the patient to a lesser extent.

Optionally these advantages can be reached in combination with each other, then, of course, to a somewhat lesser extent but nevertheless with sufficiently attractive prospects.

Preferably, to reach superior results, the images are acquired by the method of the present invention along four linear orbits which are performed in mutually transverse directions perpendicular to the patient's body.

It should be emphasized, that by the term "target organ" is meant the organ or tissue to be studied or investigated by using the method of the invention. The term "target organ" obviously encompasses a plurality of organs to be studied simultaneously and also a part of the body, like the head, the chest or the abdomen, or even the complete body of the patient.

It is further important to note, that the linear orbits must **not** necessarily be straight lines, but also encompass slightly curved lines. The expression "at least substantially straight lines" may be satisfactory in this connection. The variations of the linear orbits with respect to straight lines, however, must be small to meet the requirement, that the collimator focal line is made to travel throughout the target organ during the acquisition.

It has been observed, that the quality of the reconstructed images can further be improved, if during the acquisition the fan-beam collimator remains parallel to its initial position along each orbit. This can easily be reached by shifting the collimator during the acquisition accurately parallel to the patient's body, or vice versa.

The method according to the present invention is not restricted to the use of one gamma detector provided with a fan-beam collimator (detector-collimator combination, detector-fixed fan-beam collimator), but encompasses the use of up to four detector-collimator combinations, in particular of two and four combinations additionally. More gamma camera can be used in that case or, if desired, a two-headed or four-headed camera, i.e. a camera with two or four detector-collimator combinations. Of course, all collimators should be of the fan-beam type, focusing to a focal line. If a second detector-collimator combination is applied, this combination is used, simultaneously with and positioned opposite to the first one, sandwiching the patient in between.

If the use of four detector-collimator combinations is preferred, two couples of mutually opposite gamma detectors provided with fan-beam collimators are used simultaneously and in mutually perpendicular position, both couples sandwiching the patient in between; the images are acquired by moving each of the detector-collimator combinations along a linear orbit.

It has been observed, that by using a plurality of detector-collimator combinations, in particular two or four, according to the present invention, simultaneously following the various linear orbits, the sensitivity of the SPECT device can further be improved, resulting in still better reconstructed images.

Due to the fact that in the method of the invention the collimator focal line is made to travel throughout the target organ, so remains within the patient's body during acquisition, a fan-beam collimator or a plurality of fan-beam collimators can be used with a considerably reduced focal length, more in particular a focal length of between approx. 12 and approx. 30 cm, preferably of approx. 25 cm. As a result, the patient to be examined and also the target organ or organs can now easily be positioned within the collimator focal line where both the sensitivity and the resolution are optimal. In this pre-eminently suitable method of the invention, wherein a considerably reduced collimator focal length is used, the sensitivity can be improved with a factor of approximately 10 compared with the best actual system, if a same spatial resolution is applied. This sensitivity even further increases when the size of the studied organ decreases. In addition, the reduction of the usable transverse slice size, need to avoid image truncation, as observed in the usual SPECT technique using fan-beam collimators, is no longer present.

To improve their results, gamma cameras for SPECT imaging are often adapted to the special organs to be studied (organ-dedicated), for example, head-dedicated equipment for specific study of the head (by using an annular camera), etc. If in the method of the invention head-dedicated cameras are preferred, such cameras have only to be equipped with fan-beam collimators with a focal length of approx. 12 cm. The method of the present invention, however, gives so much better reconstructed images, that this method is well applicable for the whole body of a patient as well as for only a part of the body, e.g. the head, without adverse effects on the quality of these images. Therefore, the method of the invention can be considered as universally applicable or allround, in that fan-beam collimators with a focal length of approximately 25 cm can be used generally, i.e. both for the whole body and for organ-dedicated SPECT imaging.

It has further been found, that the distribution **A(x,y,z)** of the radioactivity in the patient's body can be computed using the following new reconstruction algorithm (this is in fact the mathematical proof that the acquired set of planar images is complete, i.e. sufficient to reconstruct the distribution activity):

$$A(x,y,z) = \frac{1}{(2\pi)^2 f} \int_{-f}^{f} dr \left( \tilde{P}_{left}(\tfrac{r}{f}x + y, r, z) + \tilde{P}_{under}(\tfrac{r}{f}y + x, r, z) + \tilde{P}_{right}(\tfrac{r}{f}x + y, r, z) + \tilde{P}_{over}(\tfrac{r}{f}y + x, r, z) \right)$$

$$\tilde{P}_{\alpha}(W,r,z) = \frac{1}{2\pi} \int_{-\infty}^{+\infty} dk \, e^{ikW} |k| \int_{-2f}^{+2f} dV \, e^{-ikV} P_{\alpha}(V,r,z)$$

wherein:

**x, y** and **z** are the orthogonal coordinates along the horizontal transverse direction, the vertical transverse direction and the longitudinal direction, respectively;

$P_{\alpha}(V,r,z)$ are the planar images pixels values, where **r** is the coordinate along the transverse direction of the detector and **V** is the detector position along the linear orbit **a**; and

**f** is the fan-beam collimator focal-length.

The limit[-f,f] in the integration dr shows that the transverse size of the detector must be greater than two times the collimator focal length, according to the above algorithm. It is also important to point out that $P_a(V,r,z)$ vanishes when the target organ does no longer intersept the collimator acceptance angle, and thus the integration dV, and as a result also the acquisition orbit range can be reduced, allowing an increasing acquisition time per planar image, i.e. an increasing sensitivity, for a same total acquisition time.

The above algorithm is the exact reconstruction of the acquired images under the assumption that the collimator resolution, the gamma attenuation and the gamma scatter can be neglected. If these effects should be taken into account, certain well-known algorithms, like EM-ML (see hereinbefore) can additionally be used for reconstruction purposes.

The invention also relates to an equipment for performing the above method of SPECT imaging according to the invention, comprising at least one gamma camera with at least one detector-fixed fan-beam collimator, and a bed for a patient to be examined in such a relative position, that the bed is surrounded by four collimator positions, essentially situated at the angular points of a square (which are only for simplicity reasons chosen to be situated over the bed (a), under the bed (b), and on both sides (c) and (d) of the bed), which positions can be occupied by said at least one collimator focusing to a focal line parallel to the bed length. The usual equipment for imaging a patient by SPECT comprises a gamma camera with one or two (two-headed) detector-fixed collimators, which follow a revolution orbit around the patient's body. The patient to be examined is fixedly positioned on a bed. During the revolution the collimator continuously points to (faces) the body of the patient and occupies successively all collimator positions of the revolution orbit, so including the above-defined four collimator positions. If a fan-bean collimator is used in this traditional revolution orbit technique, said collimator focuses in each of these positions to a focal line parallel to the axis of rotation of the gamma camera on the other side of the patient and consequently parallel to the patient's body (see hereinbefore).

According to the present invention, the equipment for performing the above method of imaging by SPECT is characterized in that:

- the bed is positioned at such a distance from the collimator positions, that in each position the collimator focal line is inside the patient's body on the bed; and
- the bed is adapted to allow movements vis-à-vis said at least one collimator in two perpendicular directions, both transverse to the bed length, viz. a sideward movement at position a or b of said at least one collimator and an up and downward movement at position c or d thereof; or, alternatively, said at least one collimator is adapted to allow movements vis-à-vis the bed in perpendicular directions, all transverse to the bed length, viz. substantially parallel to the bed surface in the position a and b, and substantially perpendicular to the bed surface in the positions c and d.

By positioning the bed at such a distance from the fan-beam collimator positions (this positioning can be adjusted by a computer, preferably by the acquisition computer), that in each of these positions the collimator focal line is inside the patient's body on the bed, the collimator focal line travels through the patient's body of the target organ therein during the acquisition by the gamma camera along linear orbits. By adapting the bed or the fan-beam collimator in such manner that it allows relative perpendicularly directed movements, as described above, images can be acquired by the gamma camera along four linear orbits performed in mutually transverse directions perpendicular to the patient's body.

The range of the relative movements of the bed vis-à-vis the collimator or collimators should preferably be at least equal to two times the transverse size of the detector or collimator, and should preferably amount to approximately 100 cm. As is already explained hereinbefore, the fan-beam collimator(s) forming part of the equipment of the invention has (have) advantageously a focal length of between approx. 12 and approx. 30 cm. If allround, i.e. not dedicated to the imaging of certain target organs or parts of the body like the head, the focal length is preferably approx. 25 cm.

It should be emphasized that by the expression "at least one" should be understood: one up to four; more in particular: one, two or four.
So the equipment according to the present invention may conveniently comprise one gamma detector provided with a fan-beam collimator. Such a detector-collimator combination is equipped in such manner that it can be moved from the above-defined position a to positions c, b and d, successively, and vice versa.
It may be of advantage, however, to include a second gamma detector provided with a fan-beam collimator into the equipment of the present invention. In that case the two detector-collimator combinations are positioned opposite to each other, sandwiching bed plus patient in between, both equipped in such manner that they can be moved from position a to position c, and from position b to position d, respectively, and vice versa.
In case one or two detector-collimator combinations are present in the equipment of the invention, the equipment is preferably so adapted that the bed is movable vis-à-vis the collimator by means of a system of motive members, preferably a combination of a horizontally shifting mobile member at the foot of the bed and a jack or moving the bed into a vertical direction. This system of motive members is explained in more detail in the Examples.

In an equally advantageous embodiment the equipment of the present invention comprises four gamma detectors with fan-beam collimators, which detector-detector combinations are so positioned that they occupy positions a, b, c and d, respectively, thereby sandwiching bed plus patient in between.
In this embodiment the four detector-collimator combinations are preferably movable vis-à-vis the bed by means of a motive system, preferably a rigid frame of four mutually perpendicular rails, positioned transversally to the bed length, along which the detector-collimator combinations can slide. This motive system is also explained in the Examples.

It is another merit of the present invention that the relative movements of the bed vis-à-vis the detector-collimator combination(s) are computer controlled (cybernation) by the gamma camera. This advance system of computer-driven detector-collimator combination(s) relative to the patient's bed, which the above-defined new algorithm is conveniently used, enables the user of the system, i.e. the personnel of the clinic or hospital, to investigate the patient full-automatically by the improved SPECT imaging technique of the invention.

## Examples

The invention will now be described in greater detail with reference to the accompanying drawings, wherein:

Figures 1 and 2 are schematic representations of the equipment according to the present invention is a suitable embodiment, Fig. 1 viewed in the longitudinal direction of the bed and
Fig. 2 viewed in a direction transverse to the bed; and
Figure 3 is also a schematic representation of the equipment of the present invention, now in another suitable embodiment, viewed in the longitudinal direction of the bed, as in Fig. 1.

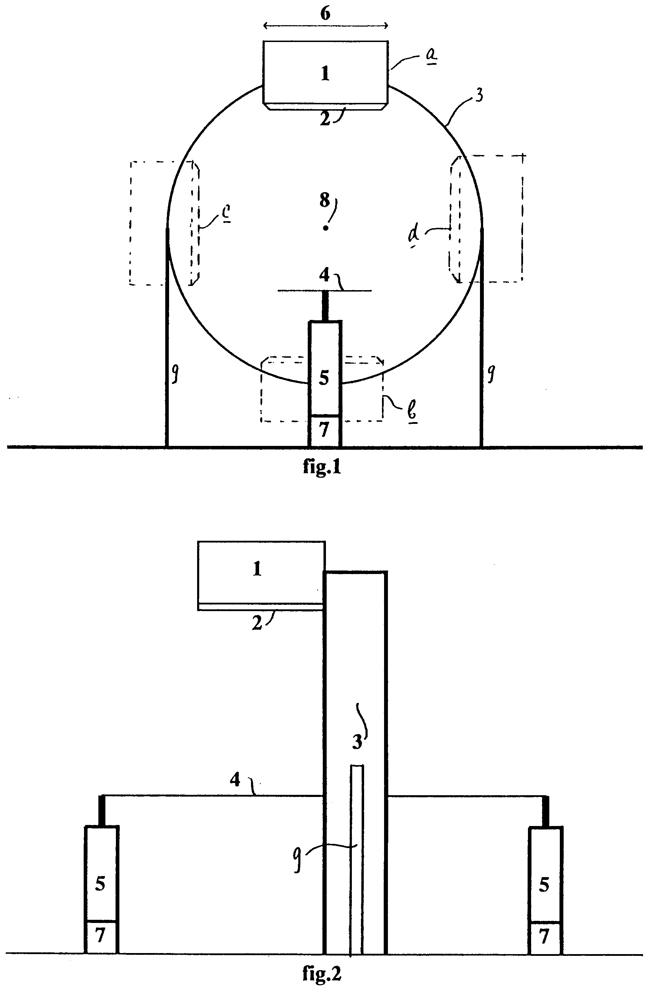

Figures 1 and 2 show a gamma detector 1 equipped with a fan-beam collimator 2 and movably attached to a circular rail 3 held by two pylons 9. The detector 1 can move along the rail, the collimator 2 always pointing to the rotation axis 8. Using a magnetic brake, the detector 1 can be positioned over, under, left and right the bed 4: positions a, b, c and d, respectively (the collimator centres are situated at the angular points of a sqare). A motor attached to the detector 1 and drawing an endless screw acting on a circular rack attached along the rail 3 can be used to move the detector-collimator combination from one position into another The bed 4 can vertically move thanks to the jacks 5, which can be constituted by a motorized endless screw acting on a rack. A crenelated plate drawing by the endless screw and inserted in an optical switch can be used to adjust the vertical position of the bed 4. This bed can also move along the left - right direction of Figure 1 (horizontal transverse direction) thanks to the mobile element 7 which can be a trolley rolling along a rail on the floor. Again a motorized endless screw acting on a rack and drawing a crenelated plate inserted in an optical switch can be used to move and adjust the transverse horizontal bed 4 position. The vertical and horizontal positioning range of the bed 4 vis-à-vis the rotation axis 8 is at least equal to two times the transverse size 6 of the detector 1. The collimator 2 focal line is parallel to the bed 4 length and goes essentially throughout the rotation axis 8. The transverse size 6 of the detector 1 is at least equal to two times the collimator focal length. The planar images are digitally acquired along four linear orbits: the bed 4 is moved into the various successive vertical positions, when the detector **1** is unmoved left or right the bed **4** (in positions c or d, respectively); the bed is moved into the various successive transverse horizontal positions, when the detector **1** is unmoved over or under the bed **4** (in positions a or b, respectively). During acquisition, the digital planar images and the vertical and horizontal digital bed **4** positions are sent to the treatment computer. The distribution of the radioactivity over the patient's body **A(x,y,z)**, wherein **x,y** and **z** are the orthogonal coordinates along the horizontal transverse direction, the vertical direction and the longitudinal direction, respectively, can be computed using the new reconstruction algorithm as disclosed hereinbefore.

A second detector - fan-beam collimator combination may be present in position b of the above equipment, movable along the rail **3** from position b to position d and vice versa, whereas the first combination is then movable from position a to position c and vice versa.

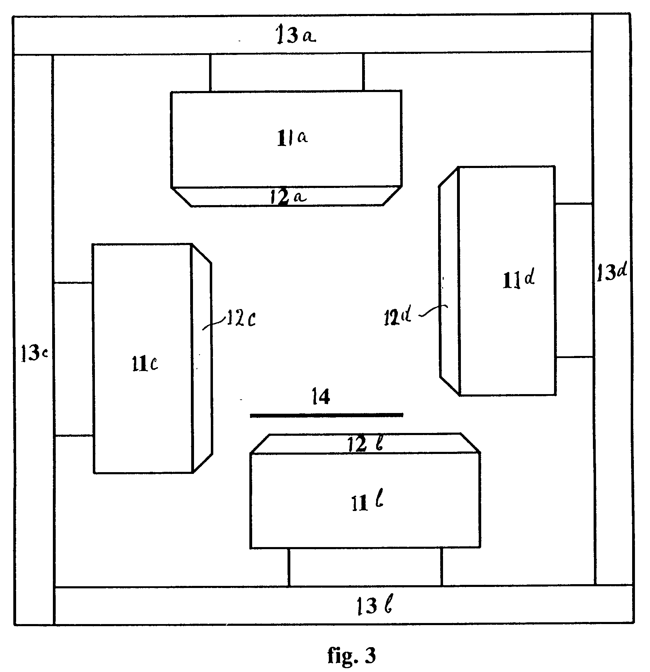

The embodiment shown in Figure 3 comprises four gamma detectors **11a**, **11b**, **11c** and **11d**, provided with fan-beam collimators **12a**, **12b**, **12c** and **12d**, situated over, under left and right the bed **14** (positions a, b, c and d, respectively). Each detector can be moved along a rail (**13a**, **13b**, **13c** and **13d**), perpendicular to the bed **14** length; the rails are attached to each other to constitute a rigid frame.

During the acquisition the detector-collimator combinations move along their rails, the bed being unmoved.

## Claims

1.   A method of imaging a target organ in patient by SPECT, by using a gamma camera having a gamma detector provided with a fan-beam collimator, focusing to a focal line parallel to the patient's body length, and by computer reconstructing the distribution of the radioactivity inside the patient's body from the acquired planar images by using certain reconstructions algorithms, said method being characterized in that the images are acquired along at least one linear orbit performed in a direction perpendicular to the patient's body, and in that the collimator focal line is made to travel throughout said target organ during the acquisition.

2.   Method as claimed in claim 1, characterized in that the images are acquired four linear orbits performed in mutually transverse directions perpendicular to the patient's body.

3.   Method as claimed in claim 1 or 2, characterized in that during the acquisition the collimator remains parallel to its initial position along each orbit.

4.   Method as claimed in any of the preceding claims, characterized in that a second gamma detector provided with a fan-beam collimator is used simultaneously with and in a position opposite to the first one, sandwiching the patient in between.

5.   Method as claimed in claim 4, characterized in that an additional couple of mutually opposite gamma detectors provided with fan-beam collimators is used simultaneously with and in a position perpendicular to the couple of claim 3, equally sandwiching the patient in between, and that the images are acquired by moving each of the detector-collimator combinations along a linear orbit.

6.   Method as claimed in any of the preceding claims, characterized in that one or more gamma detectors are used provided with one or more fan-beam collimators having a considerably reduced focal length, more in particular a focal a length of between approx. 12 and approx. 30 cm, preferably of approx. 25 cm.

7.   Method as claimed in any of the preceding claims, characterized in that, optionally in addition to certain known iterative algorithms, the following reconstruction algorithm is used:

$$A(x,y,z) = \frac{1}{(2\pi)^2 f} \int_{-f}^{f} dr \left( \tilde{P}_{left}(\frac{r}{f}x + y,r,z) + \tilde{P}_{under}(\frac{r}{f}y + x,r,z) + \tilde{P}_{right}(\frac{r}{f}x + y,r,z) + \tilde{P}_{over}(\frac{r}{f}y + x,r,z) \right)$$

$$\tilde{P}_{\alpha}(W,r,z) = \frac{1}{2\pi} \int_{-\infty}^{+\infty} dk\, e^{ikW} |k| \int_{-2f}^{+2f} dV\, e^{-ikV} P_{\alpha}(V,r,z)$$

wherein:

   **x**, **y** and **z** are the orthogonal coordinates along the horizontal transverse direction, the vertical transverse direction and the longitudinal direction, respectively;

$P_\alpha(V,r,z)$ are the planar images pixels values, where **r** is the coordinate along the transverse direction of the detector and **V** is the detector position along the linear orbit **a**; and

**f** is the fan-beam collimator focal length.

8. An equipment for performing the method as claimed in any of the preceding claims, comprising at least one gamma camera with at least one detector-fixed fan-beam collimator, and a bed for a patient to be examined in such a relative position, that the bed is surrounded by four collimator positions, essentially situated at the angular points of a square, e,g, over the bed (<u>a</u>), under the bed (<u>b</u>), and on both sides (<u>c</u>) and (<u>d</u>) of the bed, which positions can be occupied by said at least one collimator focusing to a focal line parallel to the bed length; said equipment being characterized in that:

- the bed is positioned at such a distance from the collimator positions, that in each position the collimator focal line is inside the patient's body on the bed; and
- the bed is adapted to allow movements <u>vis-à-vis</u> said at least one collimator in two perpendicular directions, both transverse to the bed length, viz. a sideward movement at position <u>a</u> or <u>b</u> of said at least one collimeter and an up and downward movement at position <u>c</u> or <u>d</u> thereof.

9. Equipment for performing the method as claimed in any of claims 1-7, comprising at least one gamma camera with at least one detector-fixed fan-beam collimator, and a bed for a patient to be examined in such a relative position, that the bed is surrounded by four collimator positions, essentially situated at the angular points of a square, e.g. over the bed (<u>a</u>), under the bed (<u>b</u>), and on both sides (<u>c</u>) and (<u>d</u>) of the bed, which positions can be occupied by said at least one collimator focusing to a focal line parallel to the bed length; said equipment being characterized in that:

- the bed is positioned at such a distance from the collimator positions, that in each position the collimator focal line is inside the patient's body on the bed; and
- said at least one collimator is adapted to allow movements <u>vis-à-vis</u> the bed in perpendicular directions, all transverse to the bed length, viz. substantially parallel to the bed surface in the positions <u>a</u> and <u>b</u>, and substantially perpendicular to the bed surface in the positions <u>c</u> and <u>d</u>.

10. Equipment as claimed in claim 8 or 9, characterized in that the range of the relative movements of the bed <u>vis-à-vis</u> said at least one collimator is at least equal to two times the transverse size of said detector or collimator.

11. Equipment as claimed in claim 10, characterized in that the range of the relative movements of the bed <u>vis-à-vis</u> said at least one collimator is approximately 100 cm.

12. Equipment as claimed in any of claims 8-11, characterized in that said at least one collimator has a focal length of between approx. 12 and approx. 30 cm, preferably of approx. 25 cm.

13. Equipment as claimed in any of claims 8-12, characterized in that the equipment comprises one gamma detector provided with a fan-beam collimator, which detector-collimator combination is equipped in such manner that it can be moved from position <u>a</u> to positions <u>c</u>, <u>b</u> and <u>d</u>, successively.

14. Equipment as claimed in any of claims 8-12, characterized in that the equipment comprises two gamma detectors provided with fan-beam collimators, which detector-collimator combinations are positioned opposite to each other, sandwiching bed plus patient in between, both equipped in such manner that they can be moved from position <u>a</u> to position <u>c</u>, and from position <u>b</u> to position <u>d</u>, respectively.

15. Equipment as claimed in any of claims 8 and 10-14 , characterized in that the bed is movable <u>vis-à-vis</u> the collimator by means of a system of motive members, preferably a combination of a horizontally shifting mobile member at the foot of the bed and a jack for moving the bed into a vertical direction.

16. Equipment as claimed in any of claims 8-12, characterized in that the equipment comprises four gamma detectors with fan-beam collimators, which detector-collimator combinations are so positioned that they occupy positions <u>a</u>, <u>b</u>, <u>c</u> and <u>d</u>, respectively, thereby sandwiching bed plus patient in between.

17. Equipment as claimed in claim 16, characterized in that the detector-collimator combinations are movable <u>vis-à-vis</u>

the bed by means of a motive system, preferably a rigid frame of four mutually perpendicular rails, positioned transversally to the bed length, along which the detector-collimator combinations can slide.

18. Equipment as claimed in any of claims 8-17, characterized in that the relative movements of the bed <u>vis-à-vis</u> the detector-collimator combination(s) are computer controlled (cybernation) by the gamma camera.

fig.1

fig.2

fig. 3